Europäisches Patentamt

European Patent Office

Office européen des brevets

(11) Publication number: **0 122 644**
**B1**

(12) **EUROPEAN PATENT SPECIFICATION**

(45) Date of publication of patent specification: **10.05.89**

(51) Int. Cl.⁴: **B 01 J 21/04, B 01 J 21/06,
B 01 J 21/14, B 01 J 23/02**

(21) Application number: **84200243.8**

(22) Date of filing: **21.02.84**

(54) **Metal hydride modified aluminium and/or silicon oxide compositions and process for their preparation.**

(30) Priority: **21.03.83 US 477185
21.03.83 US 477182
21.03.83 US 477043**

(43) Date of publication of application:
**24.10.84 Bulletin 84/43**

(45) Publication of the grant of the patent:
**10.05.89 Bulletin 89/19**

(84) Designated Contracting States:
**BE DE FR GB IT NL**

(56) References cited:
**FR-A-2 324 361
GB-A-1 217 857
US-A-3 146 209
US-A-4 293 723**

**Kirk-Othmer 3rd ed., vol. 12, p. 776
Ullmann 3rd ed., vol. 8, p. 715**

(73) Proprietor: **SHELL INTERNATIONALE
RESEARCH MAATSCHAPPIJ B.V.
Carel van Bylandtlaan 30
NL-2596 HR Den Haag (NL)**

(72) Inventor: **Lewis, Robert Malcolm
10014 Limewood Lane
Sugarland Texas 77478 (US)**
Inventor: **Slaugh, Lynn Henry
12518 Texas Army Trail
Cypress Texas 77429 (US)**

(74) Representative: **Aalbers, Onno et al
P.O. Box 302
NL-2501 CH The Hague (NL)**

Courier Press, Leamington Spa, England.

## Description

Aluminas, silicas and alumino-silicas find frequent use as catalysts and as supports for catalyst metals, particularly as supports for syngas catalysts. The support itself can in many instances modify the catalyzed reaction. Modifications of the support can thus effect the catalysts activity and selectivity as well as change the product mix. Various means have been utilized to add modifiers to supports. A frequently used method is to impregnate the support with dissolved salt or compound and then calcine the impregnated material.

In the US Patent Specification 3,146,209 is disclosed a metal oxide gel, for example, alumina gel, containing aluminium hydride bound to the surface. In the US Patent Specification 4,293,723 is disclosed an aluminous composition prepared by impregnating porous gamma alumina with aluminium hydride and subsequently heating the impregnated alumina to a temperature of from 300°C to 900°C in a non-oxidizing environment.

There have now been found aluminas, and silicas with quite different chemical and physical characteristics.

This invention relates to a process for preparing an aluminium oxide and/or silicon oxide gel composition having bonded thereto magnesium, barium or zirconium in the hydride form or if desired in the oxide form, characterized in that a substantially anhydrous aluminium and/or silicon oxide gel is contacted with powdered magnesium hybride, barium hydride or zirconium hydride in a slurry phase with the slurrying medium being an anhydrous, non-hydroxyl containing organic liquid whereby the hydride reacts with the said gel, and subsequently the gel is dried to remove the organic liquid and if desired the obtained hydride form is converted to the oxide form. Preferably the amount of magnesium hydride, barium hydride or zirconium hydride utilized is not greater than the amount needed to completely react with hydroxyl moieties present in the gel. After contact and reaction with the hydride, and after being dried to remove the solvent the gel may be calcined at elevated temperatures.

An aluminium and/or silicon oxide gel contains chemically bound water in the form of hydroxyl groups. Upon calcination at sufficiently elevated temperatures, water is given off and the gel is converted to the oxide with two hydroxyl moieties giving one molecule of water and an oxygen is attached to a metal ion.

Prior to use the oxide gels should be substantially free of absorbed water. The absorbed or free water is removed by heating the gels at temperatures ranging from 100°C to 900°C prior to contact with the hydride. Any environment that provides for drying is suitable such as air, vacuum, inert gas such as nitrogen, etc. The dried gels should be kept away from a humid atmosphere after drying. It is understood that a dried gel will still contain chemically bound water in the form of hydroxide and hydroxy-oxide.

An aluminium oxide gel is one of the substrates. This alumina can be any of the variety of available aluminas. These are commercially available as alumina gels, activated aluminas or gamma aluminas. Regarding purity of the alumina, it may be stated that small amounts of impurities are not generally detrimental.

Silica gel is also another substrate. These are readily available commercially and are essentially substantially dehydrated amorphous silica. These materials are available in various density grades, from low density with surface areas ranging from about 100—200 m²/g to regular density with surface areas up to 800 m²/g. The commercially available materials are used as dessicants, selective absorbents, catalysts and catalyst supports. Regarding purity of the silica, it may be stated that small amounts of impurities are not generally detrimental.

Other substrates are the alumino-silicates. These materials contain various mixtures of aluminium and silicon oxides. They are readily available commercially and are generally employed as cracking catalysts. Typically they contain from 50 to 95, preferably from 70 to 90 percent by weight of silica. These materials can be prepared in a conventional fashion, as for example by co-precipitation, co-gellation, or by spray drying.

In general, the compositions of the invention are prepared by contacting the substrate with magnesium hydride, barium hydride or zirconium hydride in a slurry phase and allowing the hydride to react with the substrate, which reaction is evidenced by the evolution of hydrogen gas. The temperature of contact is not critical and is generally made at room temperature, although higher or lower temperatures are not precluded. In general contact temperatures of 0°C to 100°C are utilized. After reaction, the magnesium barium or zirconium is present in and on the alumina and/or silica gel in the form of an oxide although the exact form of the oxide is not known.

The magnesium hydride used to prepare the slurry is readily available commercially. It can be prepared in various ways. It has been prepared by lithium aluminium hydride reduction of magnesium compounds and by pyrolysis of alkyl magnesiums. A preferred commercial method of preparation is by synthesis from the elements using special ball-milling techniques to provide a continuous fresh surface and careful control of temperatures of hydrogen pressure.

The barium hydride used to prepare the slurry is prepared commercially by the direct reaction of the metal and hydrogen at 300°C. The reaction is highly exothermic and goes to completion rapidly. The zirconium hydride used to prepare the slurry is readiy available commercially. It can be prepared either through direct hydriding or by reduction of zirconium oxide with calcium hydride in a hydrogen atmosphere.

2

To prepare the slurry, the hydride is ground to a fine powder, for example, less than 60, (250 μm), preferably less than 100 mesh (150 μm) and then mixed with an anhydrous, non-hydroxyl-containing organic solvent. Both water and hydroxylic materials react with the hydride and therefore contact with water, and other hydroxyl-containing materials should be avoided. However, once the compositions are prepared by reaction with the hydride, they are no longer sensitive to water and to other hydroxyl-containing materials. The inert solvent used to prepare the slurry of hydride should be a solvent that is inert to the hydride. Suitable solvents include alkanes such as hexane, cyclohexane, heptane or dodecane, ethers, such as dimethyl ether, diethyl ether or tetrahydrofuran, and ketones such as dimethyl ketone, methylisobutyl ketone or methylethyl ketone.

In general, an amount of hydride is used to react with the substrate in an amount that does exceed that amount that is needed to completely react with the hydroxyl moities present in the oxide gel substrate, i.e., an excess of hydride is not used, although lesser amounts than necessary to completely react with the substrate are frequently used. In an amount of hydride greater than that needed to completely react with the hydroxyl moieties present in the substrate is utilized, then upon completion of the reaction, excess hydride would be left on the substrate. This "free" hydride would be converted to the oxide or hydroxide upon contact with hydroxyl-containing materials or by calcination in air at elevated temperatures, but would be in a different form from the oxide prepared by the reaction of the hydride with the substrate. Thus, the use of excess hydride would provide a composition according to the invention which would also contain "free" (i.e., unreacted with the substrate) hydride, hydroxide or oxide. In general, the composition of the invention will contain from 0.001 to 50, preferably from 0.01 to 25 and most preferably from 0.1 to 10 percent by weight of magnesium, barium or zirconium, measured as the metal.

In a preferred embodiment, the compositions are prepared by adding powdered hydride to a slurry of gel particles under dry box conditions. Alternatively, the gel particles can be added to a slurry of hydride powder. The resultant mixture is stirred until reaction has ceased as indicated by the cessation of hydrogen evolution. After reaction, the compositions are filtered and dried to remove the solvent. Optionally, the compositions are frequently calcined at temperatures ranging from 200°C to 900°C prior to use as catalysts or catalyst supports.

The compositions of the invention can be utilized as catalysts and as catalyst supports. The magnesium, barium or zirconium-modified materials have different physical and chemical properties than the unmodified materials or materials modified using other magnesium, barium or zirconium compounds. For example, the compositions have physical characteristics and properties that differ from those materials prepared for example, by impregnating substrates with magnesium, barium or zirconium nitrate and calcining.

Three samples, comprising magnesium on alumina were prepared in order to demonstrate the difference. Sample 1 was prepared according to the invention, as described in Example I. Sample 2 was also prepared according to the invention as described in Example I, but was subsequently calcined at elevated temperature. Sample 3 was prepared by impregnating alumina with an aqueous solution of magnesium nitrate, drying and calcining the resultant material at elevated temperatures similar to those of Sample 2. X-Ray photoelectron spectroscopy (XPS) was used to determine the surface distribution of magnesium on the surface of the samples. The samples were prepared for analysis by grinding in an argon atmosphere, followed by mounting on polymer tape. The XPS spectra were recorded on a Varian IEE spectrometer. The relative number of atoms seen on the surface of the compositions using X-Ray photoelectric spectroscopy was determined from the core level spectra for the 1s level of magnesium and the 2s level of aluminium. Table 1 shows the results are the ratios of the relative intensities of the magnesium's 1s element line to the aluminium's 2s element line.

### TABLE 1
#### Surface atomic concentrations

|  | Mg 1s/Al 2s |
|---|---|
| Sample 1: Ex $MgH_2$, 3.3% Mg on $Al_2O_3$ | 1.6 |
| Sample 2: Ex $MgH_2$, 3.3% Mg on $Al_2O_3$ Calcined | 2.9 |
| Sample 3: Ex $Mg(NO_3)_2$ 3.5% Mg on $Al_2O_3$ Calcined | 0.15 |

As can be seen from Table 1, the compositions according to this invention have the magnesium distributed over the surface in a significantly different manner than the conventionally prepared magnesium nitrate impregnated material.

To illustrate the different physical characteristics, a composition of the invention comprising barium on silica and a comparative composition prepared by impregnating silica gel with an aqueous solution of barium nitrate and subsequently calcining, were analyzed using X-Ray photoelectron spectroscopy (XPS). The samples were prepared for analyses by grinding in an argon atmosphere, followed by mounting on polymer tape. The XPS spectra were recorded in a Varian IEE spectrometer. The relative number of atoms seen on the surface of the compositions using X-Ray photoelectron spectroscopy was determined from the core level spectra for the 3d and 4d levels of barium, the 2p level of silica and the 1s level of oxygen and are shown in Table 2.

TABLE 2

| Element and core level | from $BaH_2$/ silica gel | from $Ba(NO_3)_2$/ silica gel |
|---|---|---|
| Ba 3d | 8.6 | 2.6 |
| Ba 4d | 5.5 | 1.6 |
| Si 2p | 62 | 57 |
| O 1s | 167 | 161 |

As can be seen from Table 2, the compositions according to the invention have the barium distributed over the surface in a significantly different manner than the conventionally prepared barium nitrate impregnated material.

To illustrate the different physical characteristics, a composition of the invention comprising zirconium on silica and a comparative composition prepared by impregnating silica gel with an aqueous solution of zirconium nitrate and subsequently calcining were analyzed using X-Ray photoelectron spectroscopy (XPS). The samples were prepared for analysis by grinding in an argon atmosphere, followed by mounting on polymer tape. The XPS spectra were recorded on a Varian IEE Spectrometer. The relative number of atoms seen on the surface of the compositions was determined from core level spectra for the 3d level of zirconium, the 2p level of silica and the 1s level of oxygen and are shown in Table 3.

TABLE 3

| Element and core level | from $ZrH_2$/ silica gel | from $Zr(NO_3)_2$/ silica gel |
|---|---|---|
| Zr 3d | 0.12 | 0.63 |
| Si 2p | 88 | 55 |
| O 1s | 242 | 158 |

As can be seen from Table 3, the compositions according to the invention have the zirconium distributed over the surface in a significantly different manner than the conventionally prepared zirconium nitrate impregnated material.

Example I

The following example illustrates the preparation of the magnesium modified compositions according to the invention.

In a dry box, magnesium hydride powder (0.82 g; more than about 150 micron) was added with stirring to a slurry of calcined Kaiser KA 201 Alumina (33g 590—840 µm, 20—30 mesh) in dry tetrahydrofuran (80 ml). Stirring was continued for a period of 2 days in a sealed vessel. At the end of the 2 day period the flask was opened and the tetrahydrofuran was allowed to evaporate. The resultant composition contained about 2.5%wt magnesium measured as the metal.

A similar composition was prepared using the technique described above and Davison Grade 57 Silica instead of alumina.

Utilization of composition as catalyst support

A sample of a magnesia-silica composition prepared as described above was dried under high vacuum and pelletized to 590—840 µm size. The composition (2.6 g, 9 ml) was impregnated with a solution of ruthenium trichloride in water (0.5 g of ruthenium salt containing 47.5% ruthenium on the support.

The impregnated support was calcined in a quartz tube by heating to 250°C under nitrogen gas flow (500 ml/min) for 4 hours.

The calcined catalyst was transferred to a 1.4 cm ID high-pressure 316 stainless steel tubular reactor. Silicon carbide chips (15 ml each) were used above and below the catalyst to support it in the centre of the reactor.

The catalyst was reduced by hydrogen at 6400 kPa (63 barg) and a flow of 500 ml/min by heating the reactor over a 2 hour period to 450°C and holding at 450°C for an additional 2 hour period.

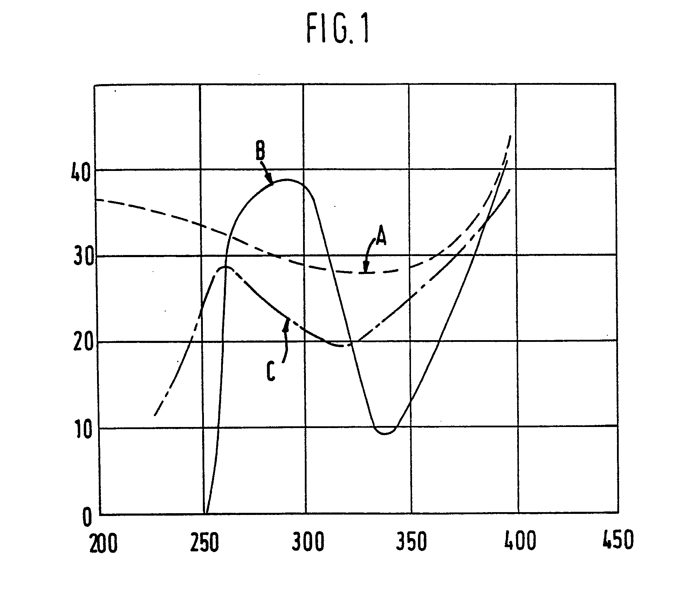

The reactor was allowed to cool to room temperature and carbon monoxide and hydrogen in a 1:1 molar ratio was passed over the catalyst at 500 ml/min (GHSV about 3000). The reactor was then heated to the desired temperature. The weight of methane in the product was determined and is plotted in Figure 1 as Curve A as a function of temperature.

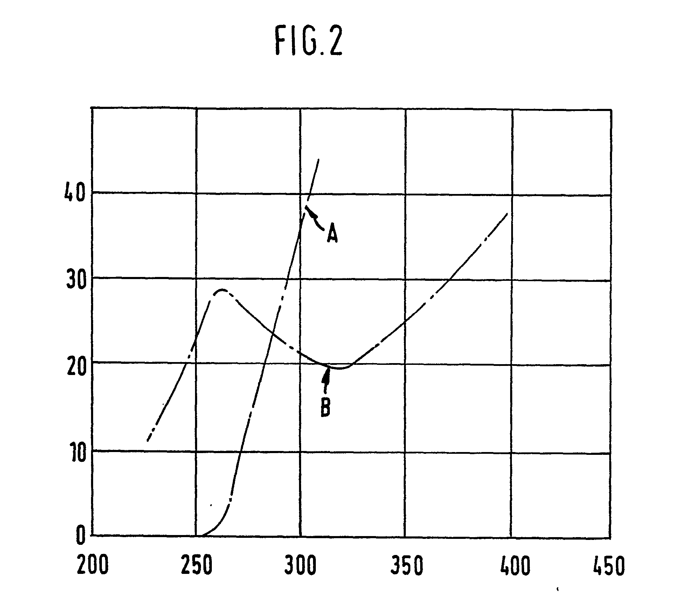

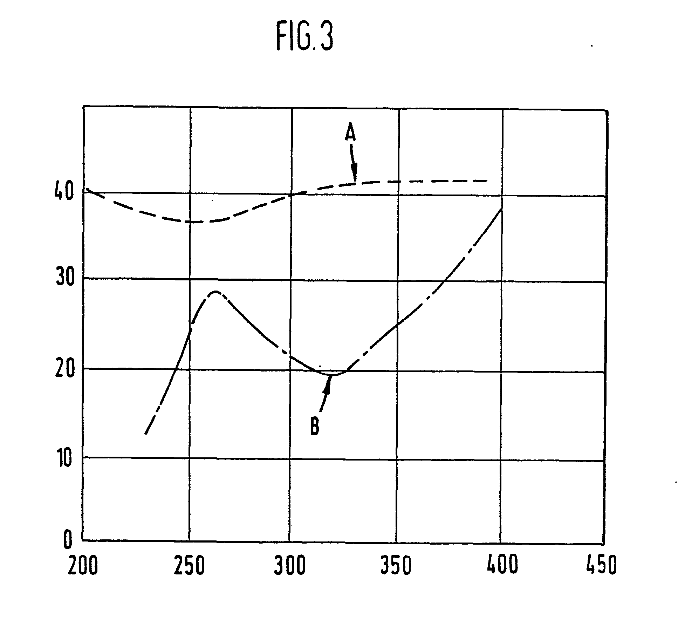

In the Figure is plotted on the vertical axis the weight percentage of methane and on the horizontal axis the temperature in degrees C. The same applies to Figures 2 and 3.

A ruthenium catalyst was made as described above but using as a support Davison Grade 57 Silica which had been impregnated with an aqueous solution of magnesium nitrate and subsequently calcined. This catalyst was tested as above and the results are shown in Figure 1 as Curve B.

The above was repeated using as a support untreated Davison Grade 57 Silica. The results are shown as Curve C in Figure 1.

As can be seen from Figure 1 the composition of the invention when used as supports provide different catalytic responses as a function of temperature than do untreated silica and silica impregnated with magnesium nitrate and subsequently calcined.

Example II

This example illustrates the preparation of the barium modified composition according to the invention.

In a dry box, barium hydride powder (0.5 g; more than about 150 micron) was added with stirring to a slurry of calcined Kaiser KA 201 Alumina (9.5 g, 590—840 µm, 20—30 mesh) in dry tetrahydrofuran (20 ml). Stirring was continued for a period of 2 days in a sealed vessel. At the end of the 2 day period the flask was opened and the tetrahydrofuran was allowed to evaporate. The resultant composition contained about 5.3 %wt barium measured as the metal.

A similar composition was prepared using the technique described above and Davison Grade 57 Silica instead of alumina.

Utilization of composition as catalyst support

A sample of a barium-modified silica composition (20 %wt BA) prepared as described above was dried under high vacuum and pelletized to 590—840 µm size. The composition (5.7 g, 9 ml) was impregnated with a solution of ruthenium trichloride in water (1.04 g of $RuCl_3 \cdot XH_2O$ in 7 ml deionized water). This gave about 8.9%wt ruthenium on the support.

The impregnated support was calcined in a quartz tube by heating to 250°C under nitrogen gas flow (500 ml/min) for 4 hours.

The calcined catalyst was transferred to a 1.4 cm ID high-pressure 316 stainless steel tubular reactor. Silicon carbide chips (18 ml each) were used above and below the catalyst to support it in the centre of the reactor.

The catalyst was reduced by hydrogen at 63 barg and a flow of 500 ml/min by heating the reactor over a 2 hour period to 450°C and holding at 450°C for an additional 2 hour period.

The reactor was allowed to cool to room temperature and carbon monoxide and hydrogen in a 1:1 molar ratio was passed over the catalyst at 500 ml/min (GHSV about 3000). The reactor was then heated to the desired temperature. The weight of methane in the product was determined and is plotted in Figure 2 as Curve A as a function of temperature.

The above was repeated using as a support untreated Davison Grade 57 Silica. The results are shown as Curve B in Figure 2.

As can be seen from Figure 2 the compositions of the invention when used as supports provide sufficient catalytic responses as a function of temperature than do unmodified silica.

The products formed by the above reactions were analyzed for paraffins and olefins by gas chromatography to provide a relatively quantitative measure of olefin isomerization. An isomerization equivalent is determined from the CG results. This involves the ratio of paraffin to olefin peak heights. As more isomerization occurs, the peak height ratio increases. This needs to be corrected, however, for the relative amounts of the olefins compared to the paraffins. This can be done by multiplying the paraffin to olefin peak height ratio by the olefin to paraffin peak area ratio. This method does not take into account the relative response factors, peak half widths, etc. However, with no isomerization occurring, the isomerization equivalent should be near one. With isomerization occurring, the number should be greater than one. The isomerization equivalents calculated for the various catalysts are tabulated in Table 4. These numbers demonstrate the remarkable effect the barium has on stopping the olefin isomerization.

TABLE 4

| Catalyst (8.9% Ru on $SiO_2$) | Paraffin/olefin peak height* | | Olefin/paraffin peak area* | | Isomerization equivalent | |
|---|---|---|---|---|---|---|
| | ~250°C | ~300°C | ~250°C | ~300°C | ~250°C | ~300°C |
| No Metal Hydride | 1.05 | 0.69 | 1.97 | 2.01 | 2.1 | 1.4 |
| 20% $BaH_2$ | 0.33 | 0.53 | 1.93 | 1.85 | 0.6 | 1.0 |

*Based on g.c. data for $C_{14}$ fraction.

Example III

This example illustrates the preparation of the zirconium modified compositions according to the invention.

In a dry box, zirconium hydride powder (0.1 g; more than about 150 micron) was added with stirring to a slurry of calcined Kaiser KA 201 Alumina (1.9 g, 590—840 µm, 20—30 mesh) in dry tetrahydrofuran (50 ml). Stirring was continued for a period of 2 days in a sealed vessel. At the end of the 2 day period the flask was opened and the tetrahydrofuran was allowed to evaporate. The resultant composition contained about 5%wt zirconium measured as the metal.

A similar composition was prepared using the technique described above and Davison Grade 57 Silica instead of alumina.

Utilisation of composition as catalyst support

A sample of a zirconia-silica composition prepared as described above containing about 10 %wt of zirconium metal was dried under high vacuum and pelletized to 590—840 µm size. The composition (5.6 g, 9 ml) was impregnated with a solution of ruthenium trichloride in water (1.9 g $RuCl_3 \cdot XH_2O$ in 9 ml of deionized water). This gave about 8.9%wt ruthenium on the support.

The impregnated support was calcined in a quartz tube by heating to 250°C under nitrogen gas flow (500 ml/min) for 4 hours.

The calcined catalyst was transferred to a 1.4 cm ID high-pressure 316 stainless steel tubular reactor. Silicon carbide chips (15 ml each) were used above and below the catalyst to support it in the centre of the reactor.

The catalyst was reduced by hydrogen at 6400 kPa (63 barg) and a flow of 500 ml/min by heating the reactor over a 2 hour period of 450°C and holding at 450°C for an additional 2 hour period.

The reactor was allowed to cool to room temperature and carbon monoxide and hydrogen in a 1:1 molar ratio was passed over the catalyst at 500 ml/min (GHSV about 300). The reactor was then heated to the desired temperature. The weight of methane in the product was determined and plotted in Figure 3 as Curve A as a function of temperature.

The above was repeated using as a support untreated Davison Grade 57 Silica. The results are shown as Curve B in Figure 3.

As can be seen from Figure 3 the compositions of the invention when used as supports provide different catalytic responses as a function of temperature than do unmodified silica.

**Claims**

1. A process for preparing an aluminium oxide and/or silicon oxide gel composition having bonded thereto magnesium, barium or zirconium, in the hydride form or if desired in the oxide form, characterized in that a substantially anhydrous aluminium and/or silicon oxide gel is contacted with powdered magnesium hydride, barium hydride or zirconium hydride in a slurry phase with slurrying medium being an anhydrous, non-hydroxyl-containing organic liquid, whereby the hydride reacts with the said gel, and subsequently the gel is dried to remove the organic liquid and if desired the obtained hydride form is converted to the oxide form.

2. A process as claimed in claim 1, characterized in that the amount of hydride utilized is not greater than the amount needed to completely react with hydroxyl moieties present in the gel.

3. A process as claimed in claims 1—2, characterized in that the hydride powder is more than 150 micrometre in size.

4. A process as claimed in claims 1—3, characterized in that the slurry liquid is tetrahydrofuran.

5. A process as claimed in claims 1—4, characterized in that the dried gel is calcined at a temperature in the range of from 200°C to 900°C.

6. A process as claimed in claims 1—5, characterized in that the magnesium, barium or zirconium in the

EP 0 122 644 B1

composition ranges from 0.001 to 50 percent by weight measured as magnesium, barium or zirconium metal.

7. A process as claimed in claim 6, characterized in that the magnesium, barium or zirconium in the composition ranges from 0.01 to 25 percent by weight measured as magnesium, barium or zirconium metal.

## Patentansprüche

1. Ein Verfahren zur Herstellung einer Aluminiumoxid- und/oder Siliciumoxid-Gelzusammensetzung, an welche Magnesium, Barium oder Zirkonium in der Hydridform oder, falls gewünscht in der Oxidform gebunden ist, dadurch gekennzeichnet, daß ein im wesentlichen wasserfreies Aluminium- und/oder Siliciumoxidgel mit pulverisiertem Magnesiumhydrid, Bariumhydrid oder Zirkoniumhydrid in einer Aufschlämmungsphase kontaktiert wird, wobei das Schlämmittel eine wasserfreie, keine Hydroxylgruppe enthaltende organische Flüssigkeit ist, wodurch das Hydrid mit einem genannten Gel reagiert, und anschließend das Gel zwecks Entfernung der organischen Flüssigkeit getrocknet wird und, falls gewünscht, die erhaltene Hydridform in die Oxidform umgewandelt wird.

2. Eine Verfahren wie in Anspruch 1 beansprucht, dadurch gekennzeichnet, daß die Menge an eingesetztem Hydrid nicht größer ist als die Menge, die für die vollständige Umsetzung mit den im Gel vorhandenen Hydroxylgruppen benötigt wird.

3. Ein Verfahren wie in den Ansprüche 1 und 2 beansprucht, dadurch gekennzeichnet, daß das Hydridpulver eine Größe von mehr als 150 µm aufweist.

4. Ein Verfahren wie in den Ansprüchen 1 bis 3 beansprucht, dadurch gekennzeichnet, daß die Aufschlämmflüssigkeit Tetrahydrofuran ist.

5. Ein Verfahren wie in den Ansprüchen 1 bis 4 beansprucht, dadurch gekennzeichnet, daß das getrocknete Gel bei einer Temperatur im Bereich von 200°C bis 900°C calziniert wird.

6. Ein Verfahren wie in den Ansprüchen 1 bis 5 beansprucht, dadurch gekennzeichnet, daß das in der Zusammensetzung enthaltene Magnesium, Barium oder Zirkonium in einer Menge im Bereich von 0,001 bis 50 Gewichtsprozent, bestimmt als Magnesium-, Barium- oder Zirkoniummetall, vorliegt.

7. Ein Verfahren wie in Anspruch 6 beansprucht, dadurch gekennzeichnet, daß das in der Zusammensetzung enthaltene Magnesium, Barium oder Zirkonium in einer Menge im Bereich von 0,01 bis 25 Gewichtsprozent, bestimmt als Magnesium-, Barium- oder Zirkoniummetall, vorliegt.

## Revendications

1. Un procédé de préparation d'une composition de gel d'oxyde d'aluminium et/ou d'oxyde de silicium auquel est lié du magnésium, du baryum ou du zirconium sous la forme d'hydrure ou si on le désire sous la forme d'oxyde, caractérisé en ce qu'un gel substantiellement anhydre d'oyxde d'aluminium et/ou d'oxyde de silicium est mis en contact avec de l'hydrure de magnésium, de l'hydrure de baryum ou de l'hydrure de zirconium pulvérisé dans une phase de bouillie, le milieu de formation de la bouillie étant un liquide organique anhydre non-hydroxylé, de façon que l'hydrure réagisse avec le gel, et ensuite le gel est séché pour élimination du liquide organique et, si on le désire, la forme hydrure obtenue est transformée en la forme oxyde.

2. Un procédé selon la revendication 1, caractérisé en ce que la quantité d'hydrure utilisée n'est pas supérieure à la quantité nécessaire pour réagir complètement avec les portions hydroxyle présentes dans le gel.

3. Un procédé selon les revendications 1—2, caractérisé en ce que la poudre d'hydrure a une grosseur de particules de plus de 150 µm.

4. Un procédé selon les revendications 1—3, caractérisé en ce que le liquide pour formation de la bouillie est du tétrahydrofuranne.

5. Un procédé selon les revendications 1—4, caractérisé en ce que le gel séché est calciné à une température comprise entre 200°C et 900°C.

6. Un procédé selon les revendications 1—5, caractérisé en ce que le magnésium, le baryum ou le zirconium est présent dans la composition à raison de 0,001 à 50% en poids, en mesurant en magnésium, baryum ou zirconium métallique.

7. Un procédé selon la revendication 6, caractérisé en ce que le magnésium, le baryum ou le zirconium est présent dans la composition à raison de 0,01 à 25% en poids, en mesurant en magnésium, baryum ou zirconium métallique.

# FIG.1

# FIG.2

# FIG.3